# FASCICULE DE BREVET EUROPEEN

(11) **EP 0 906 083 B1**
(45) Date de publication et mention de la délivrance du brevet: **23.01.2002**
(21) Numéro de dépôt: 97927253.1
(22) Date de dépôt: 06.06.1997
(51) Int. Cl.: A61K 7/06, A61K 7/50

(54) **COMPOSITIONS COSMETIQUES DETERGENTES A USAGE CAPILLAIRE ET UTILISATION**
KOSMETISCHES HAARWASCHMITTEL UND DESSEN VERWENDUNG
DETERGENT COSMETIC COMPOSITIONS FOR CAPILLARY USE AND UTILISATION THEREOF

(30) Priorité: 07.06.1996 FR 9607194
(43) Date de publication de la demande: 07.04.1999
(73) Titulaire: L'OREAL, 75008 Paris (FR)
(72) Inventeur: DECOSTER, Sandrine, F-93800 Epinay sur Seine (FR)
(74) Mandataire: Le Blainvaux Bellegarde, Françoise
(86) Numéro de dépôt international: FR9701009
(87) Numéro de publication internationale: WO9746212

(56) Documents cités:
- EP-A- 0 285 389
- EP-A- 0 400 976
- EP-A- 0 473 508
- EP-A- 0 615 742
- WO-A-92/10162
- WO-A-93/18737

## Description

La présente invention concerne de nouvelles compositions cosmétiques à propriétés améliorées destinées simultanément au nettoyage et au conditionnement des cheveux, et comprenant, dans un support cosmétiquement acceptable, une base lavante constituée de tensio-actifs à pouvoir détergent particuliers dans laquelle sont également présents à titre respectivement d'agents conditionneurs et d'agents de mise en suspension et/ou de nacrage, des silicones insolubles particulières et des esters d'acides gras et de polyols. L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensio-actifs classiques de type notamment anioniques, non-ioniques et/ou amphotères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entraîner à la longue sur la fibre capillaire des dommages plus ou moins marqués liés en particulier à l'élimination progressive des lipides ou protéines contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Parmi les agents conditionneurs les plus couramment utilisés à ce jour dans des shampooings, il convient de mentionner les silicones et/ou les dérivés de silicone, qui confèrent en effet aux cheveux lavés, secs ou mouillés, une facilité de démêlage, une douceur et un lissage nettement accrus par rapport à ce qui peut être obtenu avec les compositions nettoyantes correspondantes qui en sont exemptes. La mise en suspension de la silicone et/ou le nacrage des compositions peuvent être obtenus grace à des dérivés acylés tels que les stéarates d'éthylèneglycol ou de diéthylèneglycol (voir à cet égard EP181773 ou EP400976, EP-A-0612742, WO921062 et WO9318737).

Toutefois, et malgré les progrès réalisés récemment dans le domaine des shampooings à base de polymères de silicones, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoqués ci-avant, de meilleures performances.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en utilisant une base lavante particulière , à savoir une base lavante associant au moins un tensioactif de type anionique et au moins un tensioactif amphotère de type alkylbétaïne en C₁₀-C₁₄ comprenant à titre d'agents conditionneurs des silicones particulières convenablement sélectionnées, telles que définies ci-après, et des esters d'acides en C₁₆-C₁₈ et de polyols, à titre d'agents de mises en suspension desdites silicones et/ou d'agent de nacrage de la composition, il est possible d'obtenir des compositions détergentes présentant d'excellentes propriétés cosmétiques, en particulier au niveau de la facilité de coiffage, du maintien, de la nervosité, du lissage et de la souplesse des cheveux traités, ainsi qu'un très bon pouvoir lavant intrinsèque.

Toutes ces découvertes sont à la base de la présente invention.

Ainsi, selon la présente invention, il est maintenant proposé de nouvelles compositions capillaires détergentes et conditionnante comprenant, dans un milieu cosmétiquement acceptable, (A) une base lavante comprenant au moins un tensioactif anionique et au moins une alkylbétaïne en C₁₀-C₁₄, (B) un système conditionneur comprenant au moins une silicone insoluble choisie parmi (i) les polydialkylsiloxanes, (ii) les polydiarylsiloxanes et (iii) les polyalkylarylsiloxanes, ladite silicone étant introduite dans la composition sous forme non émulsionnée et (C) un système pour la mise en suspension de ladite silicone et/ou pour le nacrage de la composition comprenant au moins un ester d'acide en C₁₆-C₁₈ et de polyols.

L'invention a également pour objet l'utilisation en cosmétique des compositions ci-dessus pour le nettoyage et le conditionnement des cheveux.

Mais d'autres caractéristiques, aspects et avantages de l'invention apparaîtront encore plus clairement à la lecture de la description qui va suivre ainsi que des exemples concrets, mais nullement limitatifs, destinés à l'illustrer.

Comme indiqué précédemment, les éléments essentiels rentrant dans la composition des produits capillaires selon l'invention sont (A) une base lavante comprenant (i) au moins un tensioactif détergent anionique et (ii) au moins un tensioactif amphotère de type alkylbétaïne en C₁₀-C₁₄, (B) un système conditionneur comprenant la ou les silicones insolubles et (C) le ou les esters d'acides en C₁₆-C₁₈ et de polyols pour la mise en suspension de ladite ou desdites silicones et/ou pour le nacrage de la composition.

### A- BASE LAVANTE :

Les compositions conformes à l'invention comprennent nécessairement une base lavante, généralement aqueuse.

Le ou les tensioactifs formant la base lavante comprennent un ou plusieurs tensioactifs anioniques et un ou plusieurs tensioactifs amphotères alkylbétaïne en C₁₀-C₁₄.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 50 % en poids, de préférence de 10 % à 35 % en poids, et encore plus préférentiellement de 12 % à 25 % en poids, du poids total de la composition finale.

Selon une caractéristique préférée des compositions capillaires selon la présente invention, la base lavante ne contient pas d'autre tensioactifs que des tensioactifs anioniques et des tensioactifs amphotères de type alkylbétaïne en C₁₀-C₁₄.

### (i) Tensioactif(s) anionique(s) :

Leur nature ne revêt pas, dans le cadre de la présente invention, de caractère véritablement critique.
Ainsi, à titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante : 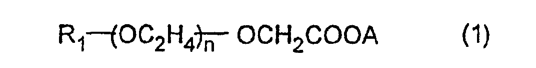 dans laquelle :
R₁ désigne un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,
A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Parmi tous ces tensioactifs anioniques, on préfère utiliser plus particulièrement les sels d'alkylsulfates et d'alkyléthersulfates, ainsi que leurs mélanges.

### (ii) Tensioactif(s) amphotère(s):

Selon l'invention, les agents tensioactifs amphotères doivent être choisis parmi les alkyl (C₁₀-C₁₄) bétaïnes de formule : 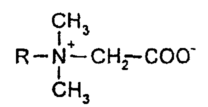 dans laquelle R désigne un radical alkyle, linéaire ou ramifié, en C₁₀-C₁₄.

En particulier, on préfère utiliser la cocoylbétaïne vendue par la société HENKEL sous la dénomination DEHYTON AB 30.

### B- SYSTEME CONDITIONNEUR

Selon une caractéristique essentielle des compositions capillaires détergentes conformes à l'invention, ces dernières contiennent en outre au moins une silicone spécifique insoluble. Cette silicone ne doit pas avoir été introduite dans la composition sous forme d'émulsion.

Selon la présente invention, par insoluble on entend insoluble dans la composition finale.

Cette silicone est choisie parmi (i) les polydialkylsiloxanes, (il) les polydiarylsiloxanes et (iii) les polyalkylarylsiloxanes.

Les radicaux alkyles comportent notamment de 1 à 10 atomes de carbone et désignent en particulier le méthyle. Les radicaux aryles désignent plus particulièrement le phényle.

Parmi les polydialkylsiloxanes, on peut citer principalement :
- les polydiméthylsiloxanes linéaires à groupements terminaux triméthylsilyle, comme par exemple, et à titre non limitatif, les huiles SILBIONE de la série 70047 commercialisées par RHONE POULENC, l'huile 47 V 500 000 de RHONE POULENC ou certaines VISCASIL de la GENERAL ELECTRIC, les FLUID DC 200 de la société DOW CORNING ;
- les polydiméthylsiloxanes linéaires à groupements terminaux hydroxydiméthylsilyle telles que les huiles de la série 48 V de RHONE POULENC.

Dans cette classe de polydialkylsiloxanes, on peut également mentionner les polydialkylsiloxanes vendus par la société GOLDSCHMIDT sous les dénominations commerciales ABILWAX 9800 et ABILWAX 9801 qui sont des polydialkyl(C₁-C₂₀)siloxanes.

Parmi les polyalkylarylsiloxanes, on peut citer les polydiméthylméthylphénylsiloxanes ou les polydiméthyldiphénylsiloxanes, linéaires ou ramifiés tels que le produit DC 556 COSMETIC GRAD FLUID de DOW CORNING.

### C- SYSTEME D'AGENT(S) DE MISE EN SUSPENSION POUR SILICONE(S) ET/OU D'AGENT(S) DE NACRAGE :

Les compositions conformes à l'invention comprennent en outre nécessairement au moins un ester d'acides en C₁₆-C₁₈ et de polyols, à titre d'agent pour la mise en suspension de la silicone rentrant dans le système conditionneur et/ou pour le nacrage de la composition.

Les mélanges d'esters d'acide en C₁₆ et de polyol et d'esters d'acides en C₁₈ et de polyol sont particulièrement préférés.

Les esters d'acides en C₁₆-C₁₈ et de polyols peuvent être choisis parmi les mono et les distéarates d'éthylèneglycol, de polyéthylèneglycol, de glycérol, de propylèneglycol et de polyglycérol, les mono et di palmitates d'éthylèneglycol, de polyéthylèneglycol, de glycérol, de propylèneglycol et de polyglycérol et leurs mélanges.
Les esters de polyéthylèneglycol et polyglycérol comportent de 2 à 150 groupements éthylèneglycol ou glycérol et de préférence de 2 à 20.

On préfère utiliser les mono- et distéarates d'éthylèneglycol, les mono- et distéarates de diéthylèneglycol, les mono- et distéarates de triéthyièneglycol, les mono- et dipalmitates d'éthylèneglycol, les mono- et les dipalmitates de glycérol

On peut par exemple utiliser le mélange (70/30 en poids) de distéarate et de dipalmitate d'éthylèneglycol vendu sous la dénomination TEGIN BL 315 par la société GOLDSCHMIDT ou le monopalmitate d'éthylèneglycol vendu sous la dénomination LANOL P par la société SEPPIC.

A titre indicatif, les compositions détergentes conformes à l'invention présentent généralement les compositions suivantes :
(i) tensio-actif(s) anionique(s) : de 5 à 50 % en poids, de préférence de 5 à 20 % en poids, par rapport au poids total de la composition détergente ;
(ii) tensio-actif(s) amphotère(s) de type alkylbétaïne : de 1 à 50 % en poids, de préférence de 1 à 20 % en poids, par rapport au poids total de la composition. De plus, la concentration en tensioactifs amphotères est généralement de 5 à 70 % en poids, et de préférence de 10 à 30 % en poids, par rapport au poids total du ou des tensio-actifs anioniques présents dans la formulation détergente ;
(iii) ester(s) d'acide en C₁₆-C₁₈ et de polyol(s) : de 0,05 % à 10 % en poids, de préférence de 0,1 % à 5 % en poids, et encore plus préférentiellement de 1 % à 4 % en poids, du poids total de la composition finale.
(iv) silicone(s) insoluble(s) non pré-émulsionnée(s) : de 0,05 % et 10 %, de préférence entre 0,1 % et 5 % et encore plus préférentiellement entre 0,2 % et 3%, par rapport au poids total de la composition.

Le véhicule, ou support, des compositions détergentes selon l'invention est de préférence de l'eau ou une solution hydroalcoolique d'un alcool inférieur tel que éthanol, isopropanol ou butanol.

Les compositions détergentes selon l'invention présentent un pH final généralement compris entre 3 et 9. De préférence, ce pH est compris entre 5 et 7. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la iriéthanolamine, l'isopropanolamine ou la propanediamine-1,3, ou encore par ajout d'un acide, de préférence un acide carboxylique tel que par exemple l'acide citrique.

Les compositions détergentes selon l'invention peuvent bien entendu contenir en outre tous les adjuvants usuels rencontrés dans le domaine des shampooings, comme par exemple des parfums, des agents conservateurs, des séquestrants, des épaississants, des adoucissants, des modificateurs de mousse, des colorants, des agents nacrants, des agents hydratants, des agents antipelliculaires ou anti-séborrhéiques, des vitamines, des filtres solaires et autres.

Bien entendu, l'homme de l'art veillera à choisir ce ou ces éventuels composés complémentaires et/ou leurs quantités de manière telle que les propriétés avantageuses attachées intrinsèquement à l'association quaternaire (tensioactif anionique + tensioactif amphotère de type alkylbétaine + ester d'acide en C₁₆-C₁₈ et de de polyol + silicone insoluble spécifique) conforme à l'invention ne soient pas, ou substantiellement pas, altérées par la ou les adjonctions envisagées.

Ces compositions peuvent se présenter sous la forme de liquides plus ou moins épaissis, de crèmes ou de gel et elles conviennent principalement au lavage, au soin et/ou le coiffage des cheveux.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Comme indiqué précédemment, les compositions conformes à l'invention confèrent aux cheveux, après rinçage, un remarquable effet traitant qui se manifeste notamment par une facilité de démêlage, de coiffage, ainsi qu'un lissage et une douceur, nettement améliorés.

L'invention a également pour objet un procédé de lavage et de conditionnement des fibres kératiniques telles que les cheveux consistant à appliquer sur lesdites fibres mouillées une quantité efficace d'une composition telle que définie ci-dessus, puis à effectuer un rinçage à l'eau après un éventuel temps de pause.

Un exemple concret, mais nullement limitatif, illustrant l'invention va maintenant être donné.

### EXEMPLE

On a réalisé une composition de shampooing contenant :
- Lauryléthersulfate de sodium
   (C12/C14 à 70/30) à 2,2 moles
   d'oxyde d'éthylène
   (MA = matière active) 14 gMA
- Cocoyl bétaine en solution aqueuse à 32%
   de matière active (DEHYTON AB 30 de HENKEL) 2,56 gMA
- Silicone insoluble (*) 2,7 g
- Distéarate et dipalmitate d'éthylèneglycol (TEGIN BL 315 de GOLDSCHMIDT) 2,5 g
- Monoisopropanolamide d'acides de coprah 0,65 g
- Acide citrique qs pH 7
- Eau déminéralisée qsp 100 g

(*) : Polydiméthylsiloxane vendu sous la dénomination de MIRASIL DM 500 000 par la Société RHONE POULENC utilisé et introduit tel quel dans la composition à préparer.

On effectue un shampooing en appliquant environ 12 g de la composition sur des cheveux préalablement mouillés. On fait mousser le shampooing puis on rince abondamment à l'eau.

Un panel d'experts a trouvé que les compositions conformes à l'invention confèrent aux cheveux, après rinçage, un remarquable effet traitant qui se manifeste notamment par une facilité de démêlage, de coiffage, ainsi qu'un lissage et une douceur remarquables des cheveux.

## Revendications

1. Compositions capillaires détergentes et conditionnantes, **caractérisées par le fait qu'**elles comprennent, dans un milieu cosmétiquement acceptable, (A) une base lavante comprenant au moins un tensioactif anionique et au moins une alkylbétaïne en C₁₀-C₁₄, (B) un système conditionneur comprenant au moins une silicone insoluble choisie parmi (i) les polydialkylsiloxanes, (ii) les polydiarylsiloxanes et (iii) les polyalkylarylsiloxanes, ladite silicone étant introduite dans la composition sous forme non émulsionnée et (C) un système pour la mise en suspension de ladite silicone et/ou pour le nacrage de la composition comprenant au moins un ester d'acide en C₁₆-C₁₈ et de polyol.

2. Compositions selon la revendication 1, **caractérisées par le fait que** le ou lesdits tensio-actifs anioniques sont présents à raison de 5 à 50 % en poids, de préférence de 5 à 20 % en poids, par rapport au poids total de la composition.

3. Compositions selon l'une quelconque des revendications précédentes, **caractérisées par le fait que** le ou lesdits tensio-actifs amphotères sont présents à raison de 1 à 50 % en poids, de préférence de 1 à 20 % en poids, par rapport au poids total de la composition.

4. Compositions selon l'une quelconque des revendications précédentes, **caractérisées par le fait que** le ou lesdits tensio-actifs amphotères sont présents à raison de 5 à 70 % en poids, de préférence de 10 à 30 % en poids, par rapport au poids total du ou des tensio-actifs anioniques.

5. Compositions selon l'une quelconque des revendications précédentes, **caractérisées par le fait que** ledit tensio-actif amphotère est la cocoylbétaine.

6. Compositions selon l'une quelconque des revendications précédentes, **caractérisées par le fait que** ledit ester d'acide en C₁₆-C₁₈ et de polyol est choisi parmi les mono et les distéarates d'éthylèneglycol, de polyéthylèneglycol, de glycérol, de propylèneglycol et de polyglycérol, les mono et dipalmitates d'éthylèneglycol, de polyéthylèneglycol, de glycérol, de propylèneglycol et de polyglycérol et leurs mélanges.

7. Compositions selon la revendication 6, **caractérisées par le fait que** ledit ester d'acide en C₁₆-C₁₈ et de polyol est choisi parmi les mono et les distéarates d'éthylèneglycol, les mono et distéarates de diéthylèneglycol, les mono et distéarates de triéthylèneglycol, les mono et les dipalmitates d'éthylèneglycol, les mono et dipalmitates de diéthylèneglycol, les mono et dipalmitates de triéthylèneglycol.

8. Compositions selon l'une quelconque des revendications précédentes, **caractérisées par le fait que** lesdits polydialkylsiloxanes sont choisis dans le groupe constitué par :
- les polydiméthylsiloxanes à groupements terminaux triméthylsilyle,
- les polydiméthylsiloxanes à groupements terminaux hydroxydiméthyl silyle.

9. Compositions selon l'une quelconque des revendications précédentes, **caractérisées par le fait que** ledit ester d'acide en C₁₆-C₁₈ et de polyol est présent à une teneur pondérale comprise entre 0,05 % et 10 % par rapport au poids total de la composition.

10. Composition selon la revendication 9, **caractérisées par le fait que** ladite teneur est comprise entre 0,1 % et 5 %.

11. Compositions selon la revendication 10, **caractérisées par le fait que** ladite teneur est comprise entre 1 % et 4 %.

12. Compositions selon l'une quelconque des revendications précédentes, **caractérisées par le fait que** la silicone insoluble est présente à une teneur pondérale comprise entre 0,05 % et 10 % par rapport au poids total de la composition.

13. Compositions selon la revendication 12, **caractérisées par le fait que** ladite teneur est comprise entre 0,1 % et 5 %.

14. Compositions selon la revendication 13, **caractérisées par le fait que** ladite teneur est comprise entre 0,2 % et 3%.

15. Compositions selon l'une quelconque des revendications précédentes, **caractérisées par le fait qu'**elles présentent un pH compris entre 3 et 9.

16. Compositions selon l'une quelconque des revendications précédentes, **caractérisées par le. fait qu'**il s'agit de compositions aqueuses ou hydroalcooliques.

17. Compositions selon l'une quelconque des revendications précédentes, **caractérisées en ce que** ladite base lavante est exempte de tensioactifs autres que des tensioactifs anioniques et des tensioactifs amphotères de type alkylbétaïne en C₁₀-C₁₄.

18. Procédé de lavage et de conditionnement des fibres kératiniques telles que les cheveux consistant à appliquer sur lesdites fibres mouillées une quantité efficace d'une composition telle que définie à l'une quelconque des revendications précédentes, puis à effectuer un rinçage à l'eau après un éventuel temps de pause.

19. Utilisation d'une composition telle définie à l'une quelconque des revendications 1 à 17 pour le nettoyage et/ou le conditionnement des cheveux.

## Patentansprüche

1. Reinigende und konditionierende Zusammensetzungen, **dadurch gekennzeichnet, daß** sie in einem kosmetisch akzeptablen Medium (A) eine reinigende Basisformulierung, die mindestens einen anionischen grenzflächenaktiven Stoff und mindestens ein C₁₀₋₁₄-Alkylbetain enthält, (B) ein Konditioniersystem, das mindestens ein unlösliches Silicon enthält, das unter (i) Polydialkylsiloxanen, (ii) Polydiarylsiloxanen und (iii) Polyalkylarylsiloxanen ausgewählt ist, wobei das Silicon in nicht emulgierter Form in die Zusammensetzung gegeben wird, und (C) ein System zur Suspendierung des Silicons und/oder für den Perlglanz der Zusammensetzung enthalten, das mindestens einen Ester einer C₁₆₋₁₈-Säure und eines Polyols enthält.

2. Zusammensetzungen nach Anspruch 1, **dadurch gekennzeichnet, daß** der oder die anionischen grenzflächenaktiven Stoffe in Mengenanteilen von 5 bis 50 Gew.-% und vorzugsweise 5 bis 20 Gew.-%, bezogen auf das Gesamtgewicht der Zusammensetzung, vorliegen.

3. Zusammensetzungen nach einem der vorhergehenden Ansprüche, **dadurch gekennzeichnet, daß** der oder die amphoteren grenzflächenaktiven Stoffe in Mengenanteilen von 1 bis 50 Gew.-% und vorzugsweise 1 bis 20 Gew.-%, bezogen auf das Gesamtgewicht der Zusammensetzung, vorliegen.

4. Zusammensetzungen nach einem der vorhergehenden Ansprüche, **dadurch gekennzeichnet, daß** der oder die amphoteren grenzflächenaktiven Stoffe in Mengenanteilen von 5 bis 70 Gew.-% und vorzugsweise 10 bis 30 Gew.-%, bezogen auf das Gesamtgewicht des oder der anionischen grenzflächenaktiven Stoffe, vorliegen.

5. Zusammensetzungen nach einem der vorhergehenden Ansprüche, **dadurch gekennzeichnet, daß** der amphotere grenzflächenaktive Stoff das Cocoylbetain ist.

6. Zusammensetzungen nach einem der vorhergehenden Ansprüche, **dadurch gekennzeichnet, daß** der Ester einer C₁₆₋₁₈-Säure und eines Polyols unter den Mono- und Distearaten von Ethylenglykol, Polyethylenglykol, Glycerin, Propylenglykol und Polyglycerin und den Mono- und Dipalmitaten von Ethylenglykol, Polyethylenglykol, Glycerin, Propylenglykol und Polyglycerin und deren Gemischen ausgewählt ist.

7. Zusammensetzungen nach Anspruch 6, **dadurch gekennzeichnet, daß** der Ester einer C₁₆₋₁₈-Säure und eines Polyols unter Ethylenglykolmono- und -distearat, Diethylenglykolmono- und -distearat, Triethylenglykolmono- und -distearat, Ethylenglykolmono- und -dipalmitat, Diethylenglykolmono- und -dipalmitat und Triethylenglykolmono- und -dipalmitat ausgewählt ist.

8. Zusammensetzungen nach einem der vorhergehenden Ansprüche, **dadurch gekennzeichnet, daß** die Polydialkylsiloxane ausgewählt sind unter:
- Polydimethylsiloxanen mit Trimethylsilylendgruppen, und
- Polydimethylsiloxanen mit Hydroxydimethylsilylendgruppen.

9. Zusammensetzungen nach einem der vorhergehenden Ansprüche, **dadurch gekennzeichnet, daß** der Ester einer C₁₆₋₁₈-Säure und eines Polyols in Mengenanteilen von 0,05 bis 10 Gew.-%, bezogen auf das Gesamtgewicht der Zusammensetzung, vorliegt.

10. Zusammensetzungen nach Anspruch 9, **dadurch gekennzeichnet, daß** der Mengenanteil im Bereich von 0,1 bis 5 % liegt.

11. Zusammensetzungen nach Anspruch 10, **dadurch gekennzeichnet, daß** der Mengenanteil im Bereich von 1 bis 4 % liegt.

12. Zusammensetzungen nach einem der vorhergehenden Ansprüche, **dadurch gekennzeichnet, daß** das unlösliche Silicon in einer Gewichtsmenge von 0,05 bis 10 Gew.-%, bezogen auf das Gesamtgewicht der Zusammensetzung, vorliegt.

13. Zusammensetzungen nach Anspruch 12, **dadurch gekennzeichnet, daß** der Mengenanteil im Bereich von 0,1 bis 5 % liegt.

14. Zusammensetzungen nach Anspruch 13, **dadurch gekennzeichnet, daß** der Mengenanteil im Bereich von 0,2 bis 3 % liegt.

15. Zusammensetzungen nach einem der vorhergehenden Ansprüche, **dadurch gekennzeichnet, daß** sie einen pH-Wert im Bereich von 3 bis 9 aufweisen.

16. Zusammensetzungen nach einem der vorhergehenden Ansprüche, **dadurch gekennzeichnet, daß** es sich um wäßrige oder wässrigalkoholische Zusammensetzungen handelt.

17. Zusammensetzungen nach einem der vorhergehenden Ansprüche, **dadurch gekennzeichnet, daß** die reinigende Basisformulierung nur anionische grenzflächenaktive Stoffe und amphotere grenzflächenaktive Stoffe vom Typ C₁₀₋₁₄-Alkylbetain und keine anderen grenzflächenaktive Stoffe enthält.

18. Verfahren zum Waschen und zum Konditionieren von Keratinfasern, wie dem Haar, das darin besteht, auf die feuchten Fasern eine wirksame Menge einer Zusammensetzung nach einem der vorhergehenden Ansprüche aufzutragen und anschließend, gegebenenfalls nach einer Einwirkzeit, mit Wasser zu spülen.

19. Verwendung einer Zusammensetzung nach einem der Ansprüche 1 bis 17 zur Reinigung und/oder zum Konditionieren der Haare.

## Claims

1. Detergent and conditioning hair compositions, **characterized in that** they comprise, in a cosmetically acceptable medium, (A) a washing base comprising at least one anionic surfactant and at least one C₁₀-C₁₄ alkylbetaine, (B) a conditioning system comprising at least one insoluble silicone chosen from (i) polydialkylsiloxanes, (ii) polydiarylsiloxanes and (iii) polyalkylarylsiloxanes, the said silicone being introduced into the composition in non-emulsified form, and (C) a system for suspending the said silicone and/or for pearling the composition, comprising at least one polyol C₁₆-C₁₈ acid ester.

2. Compositions according to Claim 1, **characterized in that** the said anionic surfactant or surfactants are present in a proportion of from 5 to 50 % by weight, preferably from 5 to 20 % by weight, relative to the total weight of the composition.

3. Compositions according to either of the preceding claims, **characterized in that** the said amphoteric surfactant or surfactants are present in a proportion of from 1 to 50 % by weight, preferably from 1 to 20 % by weight, relative to the total weight of the composition.

4. Compositions according to any one of the preceding claims, **characterized in that** the said amphoteric surfactant or surfactants are present in a proportion of from 5 to 70 % by weight, preferably from 10 to 30 % by weight, relative to the total weight of the anionic surfactant or surfactants.

5. Compositions according to any one of the preceding claims, **characterized in that** the said amphoteric surfactant is cocoylbetaine.

6. Compositions according to any one of the preceding claims, **characterized in that** the said polyol C₁₆-C₁₈ acid ester is chosen from the mono- and distearates of ethylene glycol, of polyethylene glycol, of glycerol, of propylene glycol and of polyglycerol, the mono- and dipaimitates of ethylene glycol, of polyethylene glycol, of glycerol, of propylene glycol and of polyglycerol, and mixtures thereof.

7. Compositions according to Claim 6, **characterized in that** the said polyol C₁₆-C₁₈ acid ester is chosen from the mono- and distearates of ethylene glycol, the mono- and distearates of diethylene glycol, the mono- and distearates of triethylene glycol, the mono- and dipalmitates of ethylene glycol, the mono- and dipalmitates of diethylene glycol, and the mono- and dipalmitates of triethylene glycol.

8. Compositions according to any one of the preceding claims, **characterized in that** the said polydialkylsiloxanes are chosen from the group consisting of:
- polydimethylsiloxanes containing terminal trimethylsilyl groups,
- polydimethylsiloxanes containing terminal hydroxydimethylsilyl groups.

9. Compositions according to any one of the preceding claims, **characterized in that** the said polyol C₁₆-C₁₈ acid ester is present in a weight content of between 0.05 % and 10 % relative to the total weight of the composition.

10. Composition according to Claim 9, **characterized in that** the said content is between 0.1 % and 5 %.

11. Compositions according to Claim 10, **characterized in that** the said content is between 1 % and 4 %.

12. Compositions according to any one of the preceding claims, **characterized in that** the insoluble silicone is present in a weight content of between 0.05 % and 10 % relative to the total weight of the composition.

13. Compositions according to Claim 12, **characterized in that** the said content is between 0.1 % and 5 %.

14. Compositions according to Claim 13, **characterized in that** the said content is between 0.2 % and 3 %.

15. Compositions according to any one of the preceding claims, **characterized in that** they have a pH of between 3 and 9.

16. Compositions according to any one of the preceding claims, **characterized in that** they are aqueous or aqueous-alcoholic compositions.

17. Compositions according to any one of the preceding claims, **characterized in that** the said washing base is free of surfactants other than anionic surfactants and amphoteric surfactants of C₁₀-C₁₄ alkylbetaine type.

18. Process for washing and conditioning keratin fibres such as the hair, which consists in applying an effective amount of a composition as defined in any one of the preceding claims to the said wet fibres, and then in rinsing with water after optionally leaving the composition to stand on the fibres.

19. Use of a composition as defined in any one of Claims 1 to 17 for cleaning and/or conditioning the hair.
